# EUROPÄISCHE PATENTANMELDUNG

(11) **EP 2 052 678 A1**
(43) Veröffentlichungstag der Anmeldung: **29.04.2009**
(21) Anmeldenummer: 07119134.0
(22) Anmeldetag: 24.10.2007
(51) Int. Cl.: A61B 5/00

(54) **Medizinisches System mit Verbrauchsmittel-Überwachung**

(71) Anmelder: F. Hoffmann-La Roche AG, 4070 Basel (CH); Roche Diagnostics GmbH, 68305 Mannheim (DE)
(72) Erfinder: Die Erfindernennung liegt noch nicht vor
(74) Vertreter: Hörschler, Wolfram Johannes

(57) **Zusammenfassung**

Es wird ein medizinisches System (110) vorgeschlagen, welches eine zentrale Steuereinheit (114) umfasst, die eingerichtet ist, um mindestens einen diagnostischen Messwert mittels eines Anzeigeelements (118) anzuzeigen. Das medizinische System (110) umfasst weiterhin mindestens eine invasive Einheit (120), wobei die invasive Einheit (120) mindestens ein invasives Verbrauchsmittel (132) aufweist. Das invasive Verbrauchsmittel (132) ist eingerichtet, um invasiv in ein Gewebe eines Patienten einzugreifen. Die invasive Einheit (120) weist mindestens einen berührungslos auslesbaren elektronischen Identifikator (140) zur Speicherung mindestens einer Information auf. Die zentrale Steuereinheit (114) ist eingerichtet, um die mindestens eine Information des elektronischen Identifikators (140) elektronisch auszulesen.

## Beschreibung

### Gebiet der Erfindung

Die Erfindung betrifft ein medizinisches System, welches eine zentrale Steuereinheit und eine invasive Einheit mit mindestens einem invasiven Verbrauchsmittel umfasst. Weiterhin betrifft die Erfindung ein Verfahren zur Überwachung eines medizinischen Systems. Derartige medizinische Systeme und Verfahren zur Überwachung der medizinischen Systeme werden insbesondere im Bereich der medizinischen Diagnostik und medizinischen Therapeutik eingesetzt, beispielsweise im Rahmen eines Homecare-Programmes für Diabetes-Patienten oder im Rahmen der Krankenhaus-Diagnostik oder der Therapeutik in Krankenhäusern und Pflegeheimen. Auch andere Einsatzgebiete sind möglich.

### Stand der Technik

Bei unterschiedlichen Erkrankungen müssen die Konzentrationen verschiedener Analyte in Körperflüssigkeiten der Patienten regelmäßig überwacht werden. Beispielsweise ist die Überwachung der Blutglukose-Konzentration für Diabetiker ein wesentlicher Bestandteil des Tagesablaufs. Dabei muss die Blutglukose-Konzentration in der Regel schnell und zuverlässig mehrmals am Tag bestimmt werden, um gegebenenfalls entsprechende medizinische Maßnahmen ergreifen zu können.

Neben der Diagnostik spielt auch eine entsprechende Medikation eine entscheidende Rolle. So muss beispielsweise ein Diabetiker, um Schwankungen in der Blutglukose-Konzentration auszugleichen, eine an die gemessenen Blutglukose-Konzentrationen angepasste Insulin-Medikation vornehmen.

Um den Tagesablauf der Patienten, insbesondere der Diabetiker, nicht mehr als nötig einzuschränken, werden sowohl für die Diagnostik als auch für die Medikation häufig entsprechende mobile Geräte eingesetzt, welche einfach zu transportieren und zu handhaben sein sollten, so dass die Messung bzw. die Medikation beispielsweise am Arbeitsplatz, unterwegs oder in der Freizeit erfolgen kann.

Für die Diagnostik, beispielsweise zur Bestimmung bestimmter Analyte in Körperflüssigkeiten (zum Beispiel in Blut), insbesondere zur Bestimmung von Blutglukose oder Lactat, ist die Gewinnung einer ausreichenden Menge dieser Körperflüssigkeit erforderlich. Zur Gewinnung beispielsweise einer Blutprobe oder einer anderen Probe einer Körperflüssigkeit wird meist mit einer Lanzette die Haut des Patienten an ausgewählten Körperpartien, beispielsweise an der Fingerbeere oder dem Ohrläppchen, durchstochen. Um die Blutgewinnung komfortabel zu gestalten, die Einstichtiefe zu kontrollieren und um den Schmerz zu minimieren, bieten verschiedene Hersteller Stechhilfen an, welche eine Lanzette kontrolliert und geführt in eine Hautpartie einstechen. Eine derartige Stechhilfe wird beispielsweise in EP 0 565 970 B1 beschrieben. Diese Stechhilfen können auch reversibel mechanisch mit einem zur Bestimmung der Analytkonzentration verwendeten Messgerät verbunden werden, wie dies beispielsweise in EP 1 032 307 B1 beschrieben wird. Trotz dieser mechanischen Kopplung sind die Stechhilfe und das Messgerät nach wie vor jedoch unabhängig voneinander funktionierende Einheiten und bilden zusammen ein medizinisches System.

Die Lanzetten sind in der Regel Einwegprodukte, welche beispielsweise einen scharf geschliffenen Metallstift und eine angespitzte Kunststoffhalterung umfassen, welche die Verbindung zur Stechhilfe herstellt. Auch andere Ausgestaltungen von Lanzetten sind jedoch möglich, beispielsweise Lanzetten in Form scharfer Kanten oder Dorne, welche entsprechend von der Stechhilfe bewegt werden.

Aufgrund ihrer in der Regel vergleichsweise simplen Konstruktion können Lanzetten jedoch leicht gefälscht oder nachgeahmt werden. Auch andere Verbrauchsmittel sind in vielen Fällen von Nachahmungen betroffen. Aufgrund häufig mangelhafter Passformen, minderwertigen Materialien oder mangelnder Sterilisation können gefälschte Lanzetten oder andere Verbrauchsmittel jedoch beim Einsatz erhebliche Gesundheitsgefahren und Verletzungen erzeugen. Dies gilt insbesondere für invasive Verbrauchsmittel, also Verbrauchsmittel, welche eingerichtet sind, um (wie Lanzetten) in ein Körpergewebe eines Patienten einzugreifen. Ein weiterer Nachteil derartiger nachgeahmter Verbrauchsmittel besteht darin, dass durch mangelhafte Passformen teilweise die verwendeten Peripheriegeräte beschädigt werden können. Weiterhin entstehen durch nachgeahmte Verbrauchsmittel und Fälschungen den betroffenen Unternehmen erhebliche wirtschaftliche Schäden und Imageschäden.

Die Lanzetten oder andere Arten von Verbrauchsmitteln können in einem Magazin angeordnet sein, so dass der Anwender nicht vor jeder Verwendung einer Stechhilfe die Lanzette wechseln muss, sondern nur in größeren Zeitabständen das Magazin austauscht, wenn alle darin enthaltenen Lanzetten verbraucht sind. Ein solches System wird beispielsweise in EP 1 333 756 B1 beschrieben. Ein Produktbeispiel für eine Stechhilfe mit einem Lanzettenmagazin ist die Stechhilfe "Accu-Chek Multiclix" der Firma Roche Diagnostics GmbH. Auch Lanzetten-Magazine können mit den erwähnten Konsequenzen für die betroffenen Unternehmen gefälscht werden. Lanzetten, Lanzettenmagazine und Stechhilfen sind in der Regel rein mechanische Teile ohne elektrische oder analytische Funktionen.

Ähnliche Probleme, wie sie oben für Lanzetten beschrieben sind, treten auch bei anderen Arten von Verbrauchsmitteln auf. So können grundsätzlich beispielsweise auch Verbrauchsmittel betroffen sein, welche eine analytische Funktion aufweisen (zum Beispiel Testelemente oder Subkutansensoren), Verbrauchsmittel für die Medikation (zum Beispiel Kanülen, Katheter oder ähnliches) oder andere Arten von Verbrauchsmitteln. Verbrauchsmittel allgemein werden in der Diagnostik häufig auch als "Disposables" bezeichnet.

Aus anderen Bereichen der Medizin sind zahlreiche verschiedene Vorrichtungen und Verfahren bekannt, welche die Fälschungssicherheit von Medikamenten oder Medizinprodukten gewährleisten sollen. So werden beispielsweise heute eine Vielzahl von unterschiedlichen technischen Lösungen eingesetzt, die beispielsweise Hologramme oder elektronische Identifikatoren, insbesondere Hochfrequenz-Etiketten (RFID-Etiketten), umfassen, die beispielsweise auf Medikamentenverpackungen aufgeklebt werden können. Die RFID-Technologie als ein Beispiel elektronischer Identifikatoren beinhaltet dabei die berührungslose Identifizierung mittels Radiowellen. Die Informationen eines derartigen RFID-Etiketts, welches auch als RFID-Label bezeichnet wird und welches einen elektronischen Chip enthält, werden in der Regel drahtlos über Funk mit unterschiedlichen Frequenzen zu einem Lesegerät übertragen, welches die Informationen anschließend anzeigen und/oder speichern kann. Der Einsatz von RFID-Etiketten wird beispielsweise von der amerikanischen Gesundheitsbehörde FDA zur Erhöhung der Fälschungssicherung in der Pharmabranche befürwortet.

Elektronische Identifikatoren, welche eine elektronisch auslesbare Information speichern können, sind allgemein in zahlreichen Bauformen erhältlich. Neben Bauformen, welche beispielsweise einen Silicium-Chip und eine Antenne enthalten (was üblicherweise als RFID-Etikett bezeichnet wird), sind auch RFIDs bekannt, welche auf organischer Elektronik (zum Beispiel halbleitenden oder leitfähigen Polymeren) beruhen. Weiterhin sind auch preiswerte, gedruckte Bauformen elektronischer Identifikatoren bekannt. Bei diesen, auch als "Chipless Identifiers" bekannten Bauformen, wird die Information beispielsweise in einem elektrisch leitfähigen Barcode verschlüsselt und kann berührungslos über zahlreiche Antennen in einem Lesegerät ausgelesen werden. Ein Beispiel einer derartigen Bauform ist in WO 2004/088037 A1 offenbart. Weitere Identifikatoren und Verfahren zur Informationsübertragung sind zur Übertragung von Informationen von einem medizinischen Produkt zu einem Lesegerät bekannt. So werden beispielsweise bei Systemen zur Blutzuckermessung chargenspezifische Informationen vom Testträger optisch (siehe zum Beispiel EP 1 574 855 A1) oder auf drahtlose elektronische Weise (siehe beispielsweise DE 102 37 602 A1) auf ein Blutzuckermessgerät übertragen.

Weiterhin beschreibt beispielsweise US 2006/0182656 ein Magazin, in welchem beispielsweise Testelemente gelagert werden. Das Magazin wird mit einem Barcode oder einem RFID-Etikett identifizierbar gemacht. Das Magazin, welches als Testelement-Dispenser fungiert, wird hierzu mit einem RFID-Etikett versehen, auf welchem verschiedene Informationen zu den Testelementen gespeichert und dem Messgerät zur Verfügung gestellt werden können.

EP 1 043 037 A2 beschreibt eine Injektionsvorrichtung mit einem Pen, der eine auszudrückende Spritze enthält. Die Dosiermenge beim Ausdrücken der Spritze wird mit einer Dosiermengen-Einstellvorrichtung eingestellt. Für sehbehinderte Personen ist die Einstellung der Dosiermenge sehr schwierig. Daher ist ein externes Anzeigegerät vorgesehen, das an den Pen angesetzt werden kann. Über Kontakte und Gegenkontakte wird eine Information über die Dosiermenge an das Anzeigegerät übertragen und auf einem großformatigen Display angezeigt. Dabei wird auch vorgeschlagen, den Pen mit einer von dem Anzeigegerät erkennbaren Kodierung in Form von Erhöhungen oder Vertiefungen auszustatten, damit das Anzeigegerät stets weiß, mit welchem Pen oder Pentyp es zusammenarbeitet. Die in EP 1 043 037 A2 beschriebene Injektionsvorrichtung ist jedoch sehr speziell auf ein bestimmtes System zugeschnitten und somit unflexibel gegenüber wechselnden Zusammenstellungen der einzelnen Komponenten. Für die Erkennung des Pens muss eine unmittelbare mechanische Kopplung erfolgen, und bei Verwendung eines vom Anzeigegerät nicht erkennbaren Pens ist das System nicht funktionsfähig.

In EP 1 352 611 wird ein Behältnis für Testelemente beschrieben, welches mit einem Informationschip versehen sein kann, um einem Messgerät Informationen über die Anzahl und Kalibrationsdaten von Teststreifen zu übermitteln. In diesen Systemen werden über das RFID-Etikett notwendige Informationen an das Messgerät weitergegeben, die ansonsten beispielsweise über einen sogenannten ROM-Key oder sonstige Informationsquellen oder auch manuell an das Messgerät übergeben werden müssten, um eine Auswertung der Teststreifen zu ermöglichen. Demzufolge ist das RFID-Etikett in dem Dispenser, wie er in US 2006/0182656 und EP 1 352 611 beschrieben ist, lediglich eine Variante zu bekannten Verfahren und Vorrichtungen, um dem Messgerät für die Messung benötigte Informationen zur Verfügung zu stellen, ohne die eine sinnvolle Messung oder Auswertung der Messung nicht oder nur schwer möglich wäre.

Auch aus dem Bereich anderer Arten von Verbrauchsmitteln, wie beispielsweise Lanzetten, ist die Verwendung von Identifikatoren bekannt. So beschreibt beispielsweise US 2004/0138688 eine Identifizierungsmöglichkeit für eine Lanzette, bei welcher die Identifizierung in Form eines Barcodes vorliegt. Dieser Barcode verfügt jedoch nicht über elektronische Informationen und kann somit von einem Messgerät, das nicht in unmittelbaren optischen Kontakt mit dem Verbrauchsmittel tritt, nicht identifiziert werden.

Die aus dem Stand der Technik bekannten Systeme und Verfahren zur Erhöhung der Fälschungssicherheit weisen in der Praxis einige Nachteile, Unzulänglichkeiten und Herausforderungen technischer Art auf. Die meisten Verfahren erfordern ein komplexes Lese- und Anzeigegerät, um die Echtheit der geschützten Medizinprodukte zu überprüfen. Dieses ist meist nicht mobil und/oder zu teuer, um jedem Benutzer zugänglich zu sein. Daher ist der Benutzer bei den bekannten Verfahren meist nicht in der Lage, selbständig gefälschte Medizinprodukte, wie Verbrauchsmaterialien, zu erkennen. Weiterhin setzt sich in der Regel ein medizinisches System aus einer Mehrzahl einzelner Komponenten zusammen, welche, auch wenn diese Komponenten teilweise in ein gemeinsames Gehäuse integriert oder mechanisch miteinander verbunden sein können, zumeist unabhängig voneinander ihre Funktionen ausüben. Ein Beispiel sind die genannten Stechhilfen, welche in vielen Fällen rein mechanische Funktionalität aufweisen und welche funktionell nicht in Wechselwirkung mit einem Messgerät stehen, das wiederum für eine Messung einer Analytkonzentration in einer mittels der Stechhilfe erzeugten Blutprobe genutzt wird. Ein Benutzer müsste somit, um gefälschte Verbrauchsmittel zu erkennen, auch bei den oben genannten Verfahren zur Identifikation dieser Verbrauchsmittel ein separates Lesegerät mit sich führen, welches die Identifikatoren auslesen kann. Dies ist jedoch in vielen Fällen aus Platzgründen kaum möglich und stellt zudem einen erhöhten Kostenaufwand dar.

Zudem verfügen beispielsweise rein mechanisch arbeitende Peripheriegeräte, wie zum Beispiel rein mechanische Stechhilfen, in der Regel über keine Anzeigemöglichkeit zum Anzeigen der Informationen der Identifikatoren der Verbrauchsmittel. Somit können dem Nutzer keine Informationen über diese Verbrauchsmittel, wie beispielsweise Lanzetten, zur Verfügung gestellt werden. Selbst wenn ein Peripheriegerät, wie beispielsweise eine Stechhilfe, mit einem Messgerät mit Anzeigemöglichkeit gekoppelt wird, wie dies beispielsweise bei den kommerziell erhältlichen Produkten Accu-Chek Softclix Plus und Accu-Chek Compact Plus der Fall ist, sind beide Geräte in der Regel dennoch funktionell voneinander entkoppelt, so dass der Nutzer nach wie vor mangels eindeutiger Identifikationsmerkmale der Verbrauchsmittel in der Regel keine Möglichkeit hat, Fälschungen zuverlässig zu erkennen. Insgesamt ist es somit für einen Benutzer in der Regel nicht möglich, selbst zu erkennen, ob er ein sicheres Medizinprodukt verwendet oder ob er eine gefälschte Lanzette oder ein gefälschtes Lanzettenmagazin oder andere Arten ungeeigneter Verbrauchsmaterialien einsetzt.

Weiterhin weisen aktuelle technische Lösungen zur Fälschungssicherung den Nachteil auf, dass diese in vielen Fällen selbst gefälscht werden können. Dies ist beispielsweise bei Hologrammen der Fall. Weitere Nachteile bestehen beispielsweise darin, dass in vielen Fällen separate Lesegeräte erforderlich sind.

### Aufgabe der Erfindung

Aufgabe der vorliegenden Erfindung ist es daher, ein medizinisches System und ein Verfahren zur Überwachung eines medizinischen Systems bereitzustellen, welche die Nachteile bekannter medizinischer Systeme und Verfahren zur Überwachung derartiger Systeme vermeiden. Insbesondere sollen das medizinische System und das Verfahren einem Benutzer eine selbstständige, einfache, zuverlässige und schnelle Identifikation von gefälschten Verbrauchsmitteln ermöglichen.

### Beschreibung der Erfindung

Diese Aufgabe wird durch ein medizinisches System bzw. ein Verfahren zur Überwachung eines derartigen medizinischen Systems mit den Merkmalen der unabhängigen Patentansprüche gelöst. Vorteilhafte Weiterbildungen der Erfindung, welche einzeln oder in Kombination realisierbar sind, sind in den abhängigen Ansprüchen dargestellt. Der Wortlaut sämtlicher Ansprüche wird hiermit zum Inhalt dieser Beschreibung gemacht.

Eine Idee der vorliegenden Erfindung besteht darin, bislang weitgehend unabhängig voneinander fungierende Komponenten zu einem medizinischen System zusammenzufassen, in welchem eine zentrale Steuereinheit vorhanden ist, welche die oben genannten Aufgaben der Identifikation von Verbrauchsmitteln zentral übernehmen kann. Auf diese Weise kann beispielsweise als zentrale Steuereinheit ein ohnehin in dem Gesamtsystem vorhandenes Messgerät eingesetzt werden, welches über eine eigene "Intelligenz" (beispielsweise im Rahmen eines Mikrocontrollers) verfügt und welches somit geeignet ist, auch unabhängig fungierende Peripheriegeräte, wie beispielsweise Stechhilfen, zu überwachen.

Vorgeschlagen wird dementsprechend ein medizinisches System, welches eine zentrale Steuereinheit und mindestens eine invasive Einheit umfasst. Das medizinische System kann eingerichtet sein, um beispielsweise eine oder mehrere therapeutische Aufgaben und/oder diagnostische Aufgaben und/oder Aufgaben der Probengewinnung zu übernehmen. Insbesondere kann es sich um ein medizinisches System handeln, welches zur Überwachung und Behandlung von Diabetes-Patienten einsetzbar ist, insbesondere im so genannten Home Care Bereich. Das medizinische System setzt sich also aus mehreren Komponenten zusammen, welche mindestens die zentrale Steuereinheit und mindestens eine invasive Einheit umfassen. Diese Komponenten können funktionelle unabhängig voneinander sein, können dezentralisiert unabhängig voneinander gehandhabt werden, können jedoch auch ganz oder teilweise mechanisch zusammengefasst sein, beispielsweise über ein gemeinsames Gehäuse, über Verbindungselemente oder ähnliches.

Die zentrale Steuereinheit ist eingerichtet, um mindestens einen diagnostischen Messwert mittels eines Anzeigeelements anzuzeigen. Dieser diagnostische Messwert kann beispielsweise von der zentralen Steuereinheit selbst ermittelt werden, kann jedoch auch von extern in die zentrale Steuereinheit eingegeben werden. Die zentrale Steuereinheit dient somit als Schnittstelle zwischen einem Patienten und dem medizinischen System. Besonders bevorzugt ist es dabei, wenn als zentrale Steuereinheit ein Messgerät verwendet wird, welches in einem medizinischen System (beispielsweise einem medizinischen System zur Diagnose und/oder Behandlung eines Diabetes-Patienten) ohnehin vorhanden ist. Beispielsweise kann dementsprechend die zentrale Steuereinheit mindestens eine Messfunktion zur Ermittlung eines diagnostischen Messwertes aufweisen, beispielsweise eine optische und/oder elektrochemische Messfunktion zur Ermittlung eines Blutglukosewertes, eines Lactatwertes, eines Cholesterinwertes oder einer anderen Analytkonzentration in einer Probe einer Körperflüssigkeit. Zur Ausübung dieser Messfunktion kann die zentrale Steuereinheit beispielsweise über ein oder mehrere Testelemente, beispielsweise Teststreifen, verfügen. Besonders bevorzugt umfasst diese zentrale Steuereinheit eine Datenverarbeitungselektronik, wie beispielsweise einen oder mehrere Mikrocomputer oder Microcontroller, mittels derer die Anzeige des diagnostischen Messwertes unterstützt wird und mittels derer auch Datenverarbeitungsfunktionen ausgeübt werden können.

Besonders bevorzugt ist es also, wenn die zentrale Messeinheit mindestens eine optische und/oder elektrochemische Messeinheit aufweist, welche eingerichtet ist, um mittels mindestens eines Testelements eine Konzentration eines Analyten, insbesondere eines Metaboliten, in einer Körperflüssigkeit zu ermitteln. Im Weiteren sei, ohne Beschränkung des Umfangs der Erfindung, davon ausgegangen, dass die zentrale Steuereinheit ein Blutglukosemessgerät umfasst, welches mittels eines Teststreifens oder Testbandes mit einem entsprechenden Testelement und einer optischen und/oder elektrochemischen Messeinheit eine Blutglukose-Konzentration ermitteln kann und diese Blutglukose-Konzentration mittels eines, beispielsweise als Display ausgestalteten, Anzeigeelements an einen Patienten übermitteln kann.

Neben einer derart definierten zentralen Steuereinheit, wofür, wie oben beschrieben, vorzugsweise eine ohnehin in einem medizinischen System vorhandene Einheit mit eigener "Intelligenz", d.h. beispielsweise mit mindestens einem Prozessor oder einer anderen Einheit mit Möglichkeiten zur Durchführung von Rechenoperationen, eingesetzt wird, umfasst das medizinische System weiterhin mindestens eine invasive Einheit. Diese mindestens eine invasive Einheit ist vorzugsweise funktionell unabhängig von der zentralen Steuereinheit. Unter "funktionell unabhängig" ist dabei zu verstehen, dass die jeweilige Funktion von Steuereinheit und invasiver Einheit unabhängig vom Vorhandensein der jeweils anderen Einheit ausgeübt werden kann.

Die invasive Einheit umfasst mindestens ein invasives Verbrauchsmittel. Die invasive Einheit als Ganze und/oder das invasive Verbrauchsmittel sind eingerichtet, um invasiv in ein Gewebe eines Patienten einzugreifen. Unter "Eingreifen" kann dabei eine Perforation des Gewebes, beispielsweise eine Perforation einer Hautpartie zur Gewinnung einer Probe einer Körperflüssigkeit, und/oder es kann eine vorübergehende oder dauerhafte Implantation des invasiven Verbrauchsmittels hierunter verstanden werden, wie beispielsweise die Einführung einer Kanüle oder eines subkutanen Sensors in ein Körpergewebe.

Die invasive Einheit kann vorzugsweise eine Probengewinnungsfunktion aufweisen, welche beispielsweise durch eine Lanzette realisierbar ist. Dementsprechend kann die invasive Einheit beispielsweise ein als Stechhilfe ausgestaltetes Peripheriegerät aufweisen, wobei die Stechhilfe ausgestaltet ist, um eine Hautpartie eines Patienten mittels eines als Lanzette ausgestalteten Verbrauchsmittels zu perforieren.

Alternativ oder zusätzlich kann die invasive Einheit weiterhin beispielsweise eine Medikationsfunktion aufweisen, insbesondere eine Dosierungsfunktion. Diese Medikationsfunktion kann auf verschiedene Weisen realisiert werden. So kann beispielsweise die invasive Einheit ein Peripheriegerät in Form eines Medikationsstiftes aufweisen. Gebräuchlichste Ausführungsbeispiele derartiger Medikationsstifte sind Insulin-Pens. Allgemein sind derartige Medikationsstifte ausgestaltet, um einem Patienten mittels eines als Kanüle ausgestalteten Verbrauchsmittels eine Dosis eines Medikaments zu injizieren. Beispielsweise kann diese Dosis voreingestellt werden, und die Verabreichung der Dosis kann durch manuelle Betätigung des Medikationsstiftes erfolgen, wie beispielsweise das Herunterdrücken eines Stempels.

Alternativ oder zusätzlich kann die Medikationsfunktion auch automatisiert gestaltet werden, beispielsweise mittels eines als Medikationspumpe ausgestalteten Peripheriegerätes. Gebräuchlichste Ausführungsbeispiele derartiger Medikationspumpen sind Insulinpumpen, welche einem Patienten automatisiert, beispielsweise über einen vorher definierten Zeitraum, eine eingestellte Dosis an Insulin zuführen. Allgemein ist die Medikationspumpe ausgestaltet, um mittels eines als Katheter ausgestalteten Verbrauchsmittels eine Dosis eines Medikaments zu injizieren. Das Verbrauchsmittel umfasst in diesem Fall in der Regel einen Schlauch und eine Kanüle, welche beispielsweise als Einheit oder auch getrennt ausgetauscht und entsorgt werden können.

Alternativ oder zusätzlich kann die invasive Einheit weiterhin eine analytische und/oder diagnostische Funktion umfassen. Beispielsweise kann die invasive Einheit ein als Messeinheit ausgestaltetes Peripheriegerät umfassen, wobei die Messeinheit ausgestaltet ist, um mittels eines Verbrauchsmittels, das einen in einem Körpergewebe eines Patienten eingeführten Subkutansensor umfasst, eine Konzentration eines Analyten, insbesondere eines Metaboliten, in einer Körperflüssigkeit zu ermitteln. In diesem Fall kann das Verbrauchsmittel beispielsweise neben einem austauschbaren Subkutansensor Zuleitungen, Implantationshilfen oder ähnliches umfassen. Es sei darauf hingewiesen, dass der von dieser Messeinheit gelieferte Messwert vorzugsweise unabhängig ist von der oben beschriebenen Messfunktion der zentralen Steuereinheit. Beispielsweise kann die Messeinheit ausgestaltet sein, um kontinuierlich über ein oder mehrere Tage Messungen im Körpergewebe des Patienten vorzunehmen, wohingegen die zentrale Steuereinheit für punktuelle Messungen eingerichtet ist. Beispielsweise kann die zentrale Steuereinheit mit ihrer Messfunktion eingesetzt werden, um eine Kalibrationsmessung der von einem Subkutansensor gelieferten Messwerte vorzunehmen.

Aus dieser Beschreibung wird klar, dass die zentrale Steuereinheit und die invasive Einheit vorzugsweise vollständig funktionell unabhängig ausgestaltet sind, d.h. unabhängig voneinander ihre jeweiligen Funktionen ausüben, auch wenn diese Einheiten beispielsweise mechanisch miteinander verbunden sein können. Besonders bevorzugt ist es, wenn die invasive Einheit möglichst einfach ausgestaltet ist, d.h. beispielsweise nicht über eine eigene Intelligenz oder Elektronik verfügt. Beispielsweise kann die invasive Einheit reine mechanische Funktionen (wie beispielsweise eine rein mechanisch funktionierende Stechhilfe) umfassen, welche jedoch auch beispielsweise als elektromechanische Funktionen ausgestaltet sein können. So kann beispielsweise die Stechhilfe auch elektrisch angetrieben sein, und/oder die Medikationsfunktion kann durch eine Pumpe oder einen anders gestalteten Aktuator ausgestaltet werden.

In allen beschriebenen Arten der invasiven Einheiten wird somit mindestens ein invasives Verbrauchsmittel eingesetzt. Wie eingangs beschrieben, ist dieses invasive Verbrauchsmittel ein besonders kritischer Punkt des medizinischen Systems, da aufgrund der invasiven Eigenschaften des Verbrauchsmittels dieses unmittelbar mit offenem Körpergewebe eines Patienten in Berührung kommt. Insofern machen sich bei diesem Verbrauchsmittel Sterilitätsprobleme besonders kritisch bemerkbar, welche beispielsweise dann auftreten können, wenn derartige Verbrauchsmittel unzulässig oft eingesetzt werden. Eine andere Problematik ist die bereits angesprochene Fälschungsproblematik, da beispielsweise unsachgemäß verpackte, unsachgemäß gelagerte oder unsachgemäß hergestellte Verbrauchsmittel ebenfalls zu Schädigungen des Patienten führen können, mit teilweise ernsten Konsequenzen für die Gesundheit des Patienten. Eine dritte Problematik ist das Zusammenwirken zwischen dem invasiven Verbrauchsmittel und dem Rest der invasiven Einheit, beispielsweise dem Peripheriegerät. Bei Fälschungen oder unsachgemäßer Herstellung des Verbrauchsmittels kann es, wie oben beschrieben, beispielsweise zu Schädigungen der invasiven Einheit oder anderen Arten der Fehlfunktion kommen.

Dementsprechend wird vorgeschlagen, die invasive Einheit mit mindestens einem berührungslos auslesbaren elektronischen Identifikator auszustatten. Insofern wird beispielsweise auf Aspekte der US 2004/0138688 A1 zurückgegriffen, wobei jedoch kein einfacher optischer Barcode verwendet wird, sondern elektronisch auslesbare Identifikatoren, also Identifikatoren, welche auch dann auslesbar sind, wenn kein unmittelbarer Sichtkontakt zwischen Identifikator und Auslesegerät besteht. Beispiele derartiger elektronischer Identifikatoren werden unten näher aufgeführt.

Im Unterschied zum Stand der Technik wird nunmehr jedoch kein separates Lesegerät verwendet, um die Information des elektronischen Identifikators auszulesen, sondern es wird erfindungsgemäß die zentrale Steuereinheit eingerichtet, um die mindestens eine Information des elektronischen Identifikators elektronisch auszulesen. So kann beispielsweise die zentrale Steuereinheit mit einer entsprechenden Schnittstelle zur Auslesung des elektronischen Identifikators ausgestaltet werden, beispielsweise einer Schnittstelle, welche zum Senden und/oder Empfangen elektromagnetischer Wellen, vorzugsweise im Hochfrequenzbereich, eingerichtet ist.

Damit unterscheidet sich das medizinische System gemäß der vorliegenden Erfindung grundlegend von Systemen, bei welchen elektronische Identifikatoren eingesetzt werden, um die Betriebsbereitschaft des Systems herzustellen. Im Unterschied beispielsweise zur US 2006/0182656 A1, bei welcher beim Einfügen eines Teststreifens von einem Teststreifendispenser Chargeninformationen des Teststreifens an ein Messgerät übertragen werden, ohne die das Messgerät seine Funktion nicht ausüben könnte, arbeiten im vorliegenden Fall die invasive Einheit und die zentrale Steuereinheit unabhängig voneinander. Die zentrale Steuereinheit übernimmt lediglich zusätzlich die Aufgabe, die mindestens eine Information auszulesen und, wie im Folgenden näher ausgeführt, für weitere Verwendungen bereitzuhalten. In gleicher Weise unterscheidet sich die Idee der vorliegenden Erfindung auch beispielsweise von EP 1 352 611 A1.

Der Vorteil der erfindungsgemäßen Ausgestaltung des medizinischen Systems liegt darin, dass nunmehr Informationen über die mindestens eine eingesetzte invasive Einheit zentral in einem unabhängigen Steuergerät zur Verfügung stehen. Die Übermittlung dieser mindestens einen Information erfolgt, ohne dass dabei ein Kontakt zwischen der zentralen Steuereinheit und der invasiven Einheit erforderlich ist. Die Verbrauchsmittel sind somit für Geräte vorgesehen, welche von der zentralen Steuereinheit unabhängig betreibbar sind und welche beispielsweise keine eigenen elektronischen Auslesemöglichkeiten für die elektronische Information der Identifikatoren enthalten müssen. Auf diese Weise kann zum einen auf separate Lesegeräte für elektronische Identifikatoren verzichtet werden, und die invasiven Einheiten selbst, bzw. die Peripheriegeräte, können technisch einfach und vorzugsweise ohne eigene Intelligenz gehalten werden. Weiterhin stehen dennoch Informationen über die invasiven Einheiten bzw. die invasiven Verbrauchsmittel zentral in der zentralen Steuereinheit zur Verfügung. Dies ist von besonderer Bedeutung, da das medizinische System insgesamt, wenn auch durch funktionell unabhängige Einheiten realisiert, eine gemeinsame medizinische Aufgabe erfüllen soll, wie beispielsweise die diagnostische und/oder therapeutische Betreuung eines Patienten mit einem bestimmten Krankheitsbild, insbesondere eines Diabetes-Patienten. Diese zentral zur Verfügung stehende Information kann, wie nachfolgend näher ausgeführt wird, auf verschiedene Weise nutzbringend eingesetzt werden.

So kann beispielsweise die zentrale Steuereinheit eingerichtet sein, um entsprechend der ausgelesenen Information einen akustischen Hinweis und/oder einen optischen Hinweis und/oder einen haptischen Hinweis, insbesondere eine Vibration, an den Benutzer zu übermitteln. Auf diese Weise kann beispielsweise dem Benutzer eine Information darüber zur Verfügung gestellt werden, ob ein Verbrauchsmittel in der invasiven Einheit eingefügt ist, ob dieses korrekt positioniert und/oder orientiert ist, und es kann sogar eine Positionierungshilfe zur Verfügung gestellt werden. Dies ist beispielsweise bei Patienten mit Sehbehinderungen, was bei Diabetes-Patienten häufig auftritt, von großem Vorteil.

Die Information, welche berührungslos elektronisch auslesbar ist, kann beispielsweise mindestens eine der folgenden Informationen umfassen:
- eine Information über den Hersteller der invasiven Einheit und/oder des invasiven Verbrauchsmittels;
- eine Information über ein Herstelldatum der invasiven Einheit und/oder des invasiven Verbrauchsmittels;
- eine Information über die Art der invasiven Einheit und/oder des invasiven Verbrauchsmittels, insbesondere eine Chargennummer;
- eine individuelle Seriennummer;
- eine Anzahl von in der invasiven Einheit enthaltenen invasiven Verbrauchsmitteln;
- ein Haltbarkeitsdatum der invasiven Einheit und/oder des invasiven Verbrauchsmittels.

Wie oben beschrieben, kann die invasive Einheit neben dem invasiven Verbrauchsmittel zusätzlich mindestens ein Peripheriegerät aufweisen, welches mit dem invasiven Verbrauchsmittel zusammenwirkt. Es können auch mehrere invasive Verbrauchsmittel in der invasiven Einheit zusammengefasst sein, beispielsweise in einem Verbrauchsmittelmagazin. Unter einem "Verbrauchsmittelmagazin" sind dabei sämtliche gängige Arten von Behältnissen zu verstehen, welche mehr als ein invasives Verbrauchsmittel umfassen. Beispielsweise sind hier Trommelmagazine, Stangenmagazine, Reihenmagazine, Zickzackmagazine, Bandkassetten oder ähnliches zu nennen. In einer invasiven Einheit können auch verschiedene Arten invasiver Verbrauchsmittel umfasst sein, beispielsweise verschiedene Arten von Lanzetten, oder es können invasive und nicht-invasive Verbrauchsmittel umfasst sein, beispielsweise Lanzetten und einfache Testelemente.

Wenn ein Peripheriegerät eingesetzt wird, so kann der mindestens eine elektronische Identifikator beispielsweise mit dem mindestens einen invasiven Verbrauchsmittel verbunden sein, oder kann, alternativ oder zusätzlich, auch mit dem Peripheriegerät verbunden sein. In einer bevorzugten Ausführungsform kann das invasive Verbrauchsmittel beispielsweise mindestens einen ersten berührungslos auslesbaren elektronischen Identifikator zur Speicherung mindestens einer ersten Information umfassen, und das Peripheriegerät kann mindestens einen zweiten berührungslos auslesbaren elektronischen Identifikator zur Speicherung mindestens einer zweiten Information umfassen. Auf diese Weise kann beispielsweise die Betriebssicherheit dadurch erhöht werden, dass von der zentralen Steuereinheit die erste Information und die zweite Information zeitgleich oder zeitversetzt ausgelesen werden. So kann beispielsweise ermittelt werden, ob ein korrektes Verbrauchsmittel mit einem korrekten Peripheriegerät in Zusammenwirkung verwendet wird, und es kann gegebenenfalls eine Warnung an einen Benutzer ausgegeben werden, falls dies nicht der Fall ist. Die zentrale Steuereinheit kann dementsprechend eingerichtet sein, um die mindestens eine erste Information und die mindestens eine zweite Information auszulesen. Beide Informationen können beispielsweise mit Soll-Informationen (zum Beispiel einer ersten Soll-Information und einer zweiten Soll-Information) verglichen werden.

Weitere bevorzugte Ausführungsbeispiele betreffen die Verwertung der mindestens einen Information durch die zentrale Steuereinheit. So kann beispielsweise die Information für eine spätere Auswertung gespeichert werden, wofür beispielsweise die zentrale Steuereinheit einen flüchtigen und/oder nicht-flüchtigen Datenspeicher und/oder eine Datenbank zur Verfügung stellen kann. Die mindestens eine Information kann weiterhin bei einer Fehlerdiagnose ausgewertet werden, beispielsweise im Rahmen einer Kundenreklamation. Weiterhin kann die mindestens eine Information an einen Benutzer bereitgestellt werden, beispielsweise über das oben beschriebene Anzeigeelement. Dieses mindestens eine Anzeigeelement kann beispielsweise ein oder mehrere Displays, beispielsweise Matrix-Displays, oder segmentierte Displays und/oder auch einfachere Anzeigeelemente umfassen. Beispielsweise können einfache Symbole oder Leuchtfelder oder Leuchtpunkte umfasst sein. Auch andere Arten der Anzeige sind möglich, beispielsweise - wie oben beschrieben - akustische oder haptische Signale. Beispielsweise kann, wenn die Information von einem Sollwert oder Sollbereich abweicht, eine Warnung an einen Benutzer ausgegeben werden und/oder eine Warninformation in einem Datenspeicher abgelegt werden. So kann beispielsweise ein "Flag" gesetzt werden, wenn ein ungeeignetes, d.h. beispielsweise nicht autorisiertes, fehlerhaftes oder gefälschtes Verbrauchsmittel verwendet wird. Dieses Flag kann entsprechend später ausgelesen werden. Weiterhin kann, wenn eine Abweichung von einem Sollwert oder einem Sollbereich festgestellt wird, auch aktiv mindestens eine Gerätefunktion der zentralen Steuereinheit gesperrt werden, so dass beispielsweise eine fehlerhafte Funktion des gesamten medizinischen Systems verhindert wird. Weiterhin kann auch ermittelt werden, wann das invasive Verbrauchsmittel der invasiven Einheit zuletzt gewechselt wurde. Dementsprechend können beispielsweise Warnungen ausgegeben werden, wenn der letzte Wechsel mehr als eine vorgegebene Zeitspanne zurückliegt, und/oder es können auf andere Weise Wechsel des Verbrauchsmittels initiiert werden, und/oder es können wiederum aktiv Systemfunktionen gesperrt werden. Weiterhin kann anhand der mindestens einen Information beispielsweise auch ein Hersteller der invasiven Einheit und/oder des invasiven Verbrauchsmittels ermittelt werden. Diese Herstellerinformation kann beispielsweise wiederum ausgegeben werden oder kann für eine spätere Auswertung wiederum abgespeichert werden. Verschiedene andere Möglichkeiten sind denkbar und werden im Rahmen der nachfolgenden Beschreibung eines bevorzugten Verfahrens noch näher erläutert.

Wie oben beschrieben, umfasst die zentrale Steuereinheit vorzugsweise eine Schnittstelle zum Austausch der Information mit dem Identifikator. In der Regel sind jedoch mehrere derartiger Schnittstellen erforderlich, da nicht nur mit dem mindestens einen Identifikator kommuniziert wird, sondern beispielsweise auch mit externen Datenverarbeitungsgeräten. So umfasst vorzugsweise die zentrale Steuereinheit mindestens eine erste Schnittstelle zum Datenaustausch mit einem externen Datenverarbeitungsgerät und weiterhin mindestens eine zweite Schnittstelle zum Austausch der oben beschriebenen einen Information mit dem mindestens einen Identifikator. Diese Weiterbildung der Erfindung wird der Tatsache gerecht, dass bereits heute viele zentrale Steuereinheiten, beispielsweise Blutglukose-Messgeräte, über Kommunikationsschnittstellen verfügen, mittels derer eine Kommunikation beispielsweise mit einem externen Computer möglich ist. Beispielsweise können diese Schnittstellen als drahtgebundene Schnittstellen ausgebildet sein und/oder als drahtlose Schnittstellen, wie beispielsweise Bluetooth-Schnittstellen und/oder Infrarot-Schnittstellen. Über diese erste Schnittstelle hinaus umfasst die zentrale Steuereinheit dementsprechend vorzugsweise zusätzlich die mindestens eine zweite Schnittstelle, bei welcher es sich um eine Schnittstelle für einen berührungslosen elektronischen Datenaustausch mit dem mindestens einen Identifikator handelt, welche naturgemäß an die Ausgestaltung des mindestens einen Identifikators angepasst ist.

Weitere vorteilhafte Ausgestaltungen der Erfindung beziehen sich auf die Ausgestaltung des verwendeten mindestens einen Identifikators. Vorzugsweise handelt es sich bei diesem Identifikator um einen rein passiven Identifikator, welcher keine eigene Energieversorgung umfasst. Dies kann beispielsweise dadurch realisiert werden, dass ein elektronischer Barcode verwendet wird, wie er beispielsweise in WO 2004/088037 A1 beschrieben ist. Bei diesem elektronischen Barcode werden Barcode-Streifen als elektrisch leitfähige Streifen aufgebracht und können berührungslos über Antennen in einem Lesegerät ausgelesen werden. Auch RFID-Chips verfügen in der Regel über keine eigene Energieversorgung und beziehen ihre Energie aus der empfangenen elektromagnetischen Strahlung.

Weiterhin kann auch ein veränderlicher Identifikator verwendet werden, also ein Identifikator, bei welchem die eingeschriebene Information von außen veränderbar ist. Dies kann beispielsweise bei einem elektronischen Barcode dadurch erfolgen, dass auf den Barcode selbst eingewirkt wird, indem beispielsweise einzelne Streifen des Barcodes zerstört werden. Auch andere Arten von veränderlichen Identifikatoren sind bekannt, wie beispielsweise Hochfrequenz-Identifikatoren. Bei diesen Hochfrequenz-Identifikatoren, welche beispielsweise mit einem Silicium-Chip und einer Antenne und/oder einem organischen elektronischen Schaltkreis ausgestaltet sein können, können beispielsweise durch einen entsprechenden Schreibvorgang durch Einstrahlen eines elektromagnetischen Feldes Informationen gezielt eingeschrieben werden. Zum Auslesen wird von derartigen Identifikatoren in der Regel die von außen eingestrahlte Energie genutzt, um wiederum die angeforderte Information über eine oder mehrere Antennen abzustrahlen. Derartige Hochfrequenz-Chips sind aus verschiedenen Bereichen der Technik bekannt.

Weiterhin kann der mindestens eine Identifikator derart ausgestaltet sein, dass dieser auch eine Information über eine Position und/oder Orientierung des invasiven Verbrauchsmittels innerhalb der invasiven Einheit, beispielsweise innerhalb des Peripheriegerätes, umfasst. Dies kann beispielsweise bei Verwendung eines Identifikators an einem Peripheriegerät und durch Verwendung von Identifikatoren an den Verbrauchsmitteln realisiert werden, indem die relative Position der Identifikatoren zueinander bestimmt wird. Beispiele dieser Ausgestaltung werden nachfolgend näher genannt.

Neben dem medizinischen System einer der oben beschriebenen Ausführungsformen wird weiterhin ein Verfahren zur Überwachung eines medizinischen Systems vorgeschlagen.

Bei dem medizinischen System soll es sich wiederum um ein medizinisches System handeln, welches vorzugsweise insofern in sich geschlossen ist, dass es der Überwachung und/oder Diagnose und/oder Therapie eines oder mehrerer geschlossener Krankheitsbilder dient, wie beispielsweise der Betreuung von Diabetes-Patienten. Insbesondere kann es sich bei diesem medizinischen System um ein medizinisches System gemäß einer der oben beschriebenen Ausführungsformen handeln, so dass bezüglich bevorzugter Ausführungsbeispiele und Definitionen von Systemkomponenten beispielsweise auf die obige Beschreibung verwiesen werden kann.

Wiederum umfasst das medizinische System eine zentrale Steuereinheit, welche eingerichtet ist, um mindestens einen diagnostischen Messwert mittels mindestens eines Anzeigeelements anzuzeigen, sowie mindestens eine invasive Einheit mit mindestens einem invasiven Verbrauchsmittel, welches invasiv in ein Gewebe eines Patienten eingreifen kann. Die invasive Einheit umfasst wiederum mindestens einen berührungslos auslesbaren elektronischen Identifikator zur Speicherung mindestens einer Information. Das Verfahren ist derart eingerichtet, dass die zentrale Steuereinheit die mindestens eine Information des elektrischen Identifikators ausliest.

Das Verfahren in seinen bevorzugten Ausführungsvarianten bezieht sich insbesondere darauf, wie die mindestens eine Information des mindestens einen Identifikators ausgewertet wird. Im Nachfolgenden werden die verschiedenen, auch in Kombination einsetzbaren Möglichkeiten beschrieben, wobei jeweils die Betonung auf der Auswertung liegt. Natürlich können die ausgewerteten Informationen auch in einem oder mehreren Datenspeichern und/oder Datenbanken abgelegt werden. Für eine spätere Auswertung können diese einem Benutzer beispielsweise optisch und/oder akustisch und/oder haptisch übermittelt werden, und/oder können an einen externen Computer übermittelt werden (beispielsweise einen Arztcomputer oder einen Firmencomputer des Herstellers bei einer Mängeldiagnose oder Fehldiagnose) oder können auf andere Weise weiter verwertet werden.

So kann beispielsweise von der zentralen Steuereinheit anhand der Information ermittelt werden, ob das invasive Verbrauchsmittel bereits zuvor in dem medizinischen System, beispielsweise in Kombination mit einem Messgerät, verwendet wurde. Diese Information kann mehrfach benutzt werden, beispielsweise um einem Patienten beim erstmaligen Gebrauch Produktinformationen oder Gebrauchsinformationen zur Verfügung zu stellen. Alternativ oder zusätzlich kann beispielsweise auch eine Mehrfachnutzung vermieden werden, um Sterilitätsprobleme zu vermeiden.

Alternativ oder zusätzlich kann von der zentralen Steuereinheit anhand der übertragenen Information auch ermittelt werden, ob ein Haltbarkeitsdatum der invasiven Einheit und/oder des invasiven Verbrauchsmittels abgelaufen ist. In Reaktion auf diese Ermittlung kann beispielsweise wiederum eine Warnung an einen Benutzer ausgegeben werden, oder es kann, in diesem wie in anderen Fällen, auch eine Aktion ausgelöst werden, wie beispielsweise die Sperrung einer Systemfunktion des medizinischen Systems. In diesem wie in anderen Fällen kann die Information auch für eine spätere Auswertung in einem oder mehreren Datenspeichern, zum Beispiel flüchtigen oder nicht-flüchtigen Datenspeichern, der zentralen Steuereinheit gespeichert werden. Diese Information steht beispielsweise später für eine weitere Auswertung, Darstellung, Fehlerdiagnose oder als Zusatzinformation für eine ärztliche Diagnose zur Verfügung. Die Information kann insbesondere im Rahmen einer Fehlerdiagnose und/oder einer Bearbeitung einer Reklamation ausgewertet werden.

Alternativ oder zusätzlich kann das Verfahren weiterhin derart ausgestaltet sein, dass von der zentralen Steuereinheit anhand der Information ermittelt wird, ob die invasive Einheit und/oder das invasive Verbrauchsmittel für die Verwendung in dem medizinischen System geeignet ist bzw. sind. Auf diese Weise kann beispielsweise erkannt werden, ob es sich um ein vom Hersteller des Systems oder einzelner Systemkomponenten (wie zum Beispiel Peripheriegeräten) autorisiertes Verbrauchsmaterial handelt oder um Fälschungen bzw. Nachahmungen. Diese Funktionalitätsoption hat, wie oben beschrieben, nicht nur erhebliche wirtschaftliche Bedeutung für die Systemhersteller, sondern kann auch erheblich dazu beitragen, Fehlfunktionen oder Zerstörungen des medizinischen Gerätes zu vermeiden, falsche diagnostische Ergebnisse zu verhindern oder gar Infektionen oder Verletzungen durch unsachgemäß hergestellte und/oder gelagerte Verbrauchsmittel zu vermeiden. Entsprechend kann wiederum diese Information rein passiv in der zentralen Steuereinheit gespeichert werden und/oder kann für einen Patienten oder einen Arzt dargestellt werden und/oder kann für eine spätere Auswertung bereitgehalten werden, und/oder es können auch aktiv Systemfunktionen beispielsweise gesperrt werden, um Fehlfunktionen von vorneherein zu vermeiden.

Weiterhin kann das Verfahren auch derart ausgestaltet sein, dass die zentrale Steuereinheit anhand der Information ermittelt, wie viele invasive Verbrauchsmittel die invasive Einheit umfasst. Beispielsweise kann auf diese Weise eine Gesamtanzahl von Lanzetten in einem Magazin der invasiven Einheit ermittelt werden. Dies kann beispielsweise einem Benutzer angezeigt werden. Auch die Anzahl unbenutzter invasiver Verbrauchsmittel, wie beispielsweise die Anzahl unbenutzter Lanzetten, kann ermittelt werden. Dies kann beispielsweise - wie oben beschrieben - dadurch erfolgen, dass ein veränderlicher Identifikator verwendet wird, wobei beispielsweise bei jeder Verwendung eines invasiven Verbrauchsmittels der Identifikator durch die zentrale Steuereinheit und/oder durch die invasive Einheit selbst, beispielsweise durch ein Peripheriegerät, verändert wird. Im ersteren Fall kann beispielsweise eine in einem Hochfrequenz-Chip gespeicherte Information verändert werden, im letzteren Fall kann beispielsweise ein elektronischer Barcode mechanisch verändert werden, beispielsweise durch ein Entfernen oder Hinzufügen einzelner Striche oder anders gestalteter Segmente dieses Barcodes.

Weiterhin kann von der zentralen Steuereinheit ermittelt werden, ob das invasive Verbrauchsmittel bereits verwendet worden ist. Ist dies der Fall, so kann ein Benutzer zur Verwendung eines neuen invasiven Verbrauchsmittels aufgefordert werden und/oder es kann bewirkt werden, dass die invasive Einheit ein neues invasives Verbrauchsmittel verwendet. Wiederum kann somit die ausgewertete Information rein passiv verwendet werden, also beispielsweise abgespeichert werden, kann einem Benutzer durch entsprechende Anzeige übermittelt werden, oder es kann eine aktive Systemfunktion ausgelöst oder gesperrt werden.

Ist der Identifikator derart ausgelegt, dass die in diesem Identifikator gespeicherte Information geändert werden kann, beispielsweise durch die zentrale Steuereinheit und/oder ein Peripheriegerät, so kann auch eine Information über die Identität der zentralen Steuereinheit in dem Identifikator gespeichert werden. Auf diese Weise kann beispielsweise nach Erkennung eines Identifikators durch die zentrale Steuereinheit, beispielsweise ein Messgerät, eine Seriennummer der zentralen Steuereinheit beziehungsweise des Messgerätes in diesem Identifikator gespeichert werden. Wird anschließend diese Seriennummer beziehungsweise anders gestaltete Information über die Identität der zentralen Steuereinheit von der gleichen zentralen Steuereinheit oder einer anderen später verwendeten zentralen Steuereinheit eingelesen, so kann jedes der Messgeräte erkennen, dass es sich vorliegend um eine gebrauchte invasive Einheit bzw. ein gebrauchtes invasives Verbrauchsmittel handelt. Diese Zusatzinformation kann beispielsweise wiederum gespeichert und/oder angezeigt werden und/oder es können bestimmte Aktionen ausgelöst werden, wie zum Beispiel eine Sperrung von Systemfunktionen, wenn nicht ein vom Hersteller des medizinischen Systems autorisiertes Verbrauchsmittel eingelegt wurde.

Weiterhin kann, insbesondere wenn ein positionssensitiver Identifikator verwendet wird, auch erkannt werden, ob das invasive Verbrauchsmittel in der invasiven Einheit korrekt positioniert ist. Hierbei kann sogar über die zentrale Steuereinheit eine Benutzerführung dahingehend erfolgen, dass Signaltöne und/oder Vibrationen der zentralen Steuereinheit ausgegeben werden, während das Verbrauchsmittel in ein Peripheriegerät eingelegt wird. Diese Signaltöne oder Vibrationen können sich beispielsweise in Lautstärke oder Frequenz verändern, wenn ein sehbehinderter Nutzer das Verbrauchsmittel in das Peripheriegerät einlegt, so dass dieser Vorgang in der Art einer Tonpeilung unterstützt werden kann. Alternativ oder zusätzlich kann auch eine Bestätigung angezeigt werden, dass das Verbrauchsmaterial korrekt eingelegt wurde. In einem weiteren Aspekt des Verfahrens wird vorzugsweise von der zentralen Steuereinheit anhand der Information ermittelt, wann der letzte Wechsel des invasiven Verbrauchsmittels stattgefunden hat. Das Datum des letzten Wechsels kann beispielsweise wiederum gespeichert werden für eine spätere Auswertung und/oder kann an einen Benutzer ausgegeben werden. Weiterhin können auch Aktionen ausgelöst werden, wie beispielsweise eine Warnfunktion, wenn ein Wechsel eines Verbrauchsmittels erforderlich ist und/oder eine Blockierfunktion, wenn der letzte Wechsel mehr als eine vorgegebene Zeitspanne zurückliegt. Prinzipiell wäre auch die Anzahl der Verwendungen der verwendeten Verbrauchsmittel speicherbar.

Weiterhin kann, wenn das invasive Verbrauchsmittel mindestens eine Lanzette umfasst, welche mit einem Peripheriegerät in Form einer Stechhilfe zusammenwirkt, von der zentralen Steuereinheit anhand der Information auch ermittelt werden, welche Einstechtiefe an der Stechhilfe eingestellt ist. Dies kann beispielsweise dadurch realisiert werden, dass der Identifikator wiederum als veränderlicher Identifikator ausgestaltet ist, wobei eine Veränderung der Einstechtiefe eine Veränderung der in dem Identifikator gespeicherten, auslesbaren Information umfasst. Die Einstechtiefe kann beispielsweise angezeigt, abgespeichert oder anderweitig genutzt werden.

Es sei darauf hingewiesen, dass das oben beschriebene medizinische System vorzugsweise eingerichtet sein kann, um das dargestellte Verfahren in einer der beschriebenen Ausführungsvarianten zu unterstützen. So kann beispielsweise die zentrale Steuereinheit eingerichtet sein, um das Verfahren in einer oder mehrerer der dargestellten Varianten durchzuführen. Beispielsweise kann die zentrale Steuereinheit zu diesem Zweck - wie oben beschrieben - einen oder mehrere Mikrocomputer umfassen, welche beispielsweise durch entsprechende programmtechnische Einrichtungen und/oder Software die Ermittlung und/oder Speicherung und/oder Auswertung einer der genannten Zusatzinformationen unterstützen und/oder eingerichtet sind, um entsprechend dieser Zusatzinformationen weitere Aktionen vorzunehmen, beispielsweise die oben beschriebene Sperrung einer oder mehrerer Systemfunktionen des medizinischen Systems.

Für den Hersteller des medizinischen Systems oder einzelner Komponenten des medizinischen Systems ergeben sich aus dem oben beschriebenen Verfahren und dem medizinischen System in einer der beschriebenen Ausführungsformen insbesondere die Vorteile, dass die Patienten die Verbrauchsmittel rechtzeitig auswechseln können, da sie beispielsweise durch die zentrale Steuereinheit an das Datum des letzten Wechsels erinnert werden. Dies kann von wirtschaftlicher Bedeutung sein, da es sich bei Peripheriegeräten und Messgeräten in der Regel um Systemkomponenten handelt, welche nicht kostendeckend veräußert werden, wohingegen das Verbrauchsmaterial die eigentlich wirtschaftlich interessanten Komponenten darstellen. Weiterhin werden durch das Verfahren und das vorgeschlagene System Alleinstellungsmerkmale gegenüber möglichen Konkurrenten geschaffen. Zudem wird die Nachahmung von Verbrauchsmitteln, insbesondere Lanzetten, deutlich erschwert, da die Kunden nunmehr in die Lage versetzt werden können, nicht autorisierte Verbrauchsmittel selbständig zu erkennen. Hierdurch kann ein Imageschaden, welcher durch Fälschungen auftreten kann, vermieden oder reduziert werden.

Für den Patienten ergeben sich insbesondere die Vorteile, dass er durch beispielsweise Anzeige des Datums des letzten Wechsels des Verbrauchsmittels an einen Zeitpunkt eines Verbrauchsmittelwechsels erinnert werden kann. Dies steigert die Benutzerfreundlichkeit des medizinischen Systems. Weiterhin kann er, beispielsweise bei einer Blutzuckermessung, auf einen Blick erkennen, wie viele Verbrauchsmittel, beispielsweise Lanzetten, ihm für weitere Messungen noch zur Verfügung stehen. Auch die Erkennung, dass autorisierte oder nicht autorisierte Verbrauchsmittel verwendet werden, gibt dem Patienten Sicherheit. Zudem bietet die Möglichkeit der Erkennung, ob Verbrauchsmittel richtig in Peripheriegeräte eingelegt wurden, eine zusätzliche Betriebssicherheit. Zum Schutz des Patienten kann zudem bei Bedarf, zum Beispiel in einem Klinikmodus, sichergestellt werden, dass ein Verbrauchsmittel - beispielsweise eine Lanzette - nur einmal verwendet werden kann.

### Ausführungsbeispiele

Weitere Einzelheiten und Merkmale der Erfindung ergeben sich aus der nachfolgenden Beschreibung von bevorzugten Ausführungsbeispielen in Verbindung mit den Unteransprüchen. Hierbei können die jeweiligen Merkmale sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktionen einander entsprechende Elemente.

Im Einzelnen zeigt:
- Figur 1: ein bevorzugtes Ausführungsbeispiel eines einfachen medizinischen Systems für die Diabetes-Kontrolle;
- Figur 2: eine Prinzipskizze eines erweiterten medizinischen Systems mit einer Mehrzahl invasiver Einheiten;
- Figur 3: ein mögliches Ausführungsbeispiel der Anbringung eines Identifikators an einem Verbrauchsmittel;
- Figur 4: ein zu Figur 3 alternatives Ausführungsbeispiel der Anbringung eines Identifikators an einem Verbrauchsmittel;
- Figuren 5A und 5B: ein erstes Ausführungsbeispiel einer Realisierung eines positionssensitiven Identifikators; und
- Figur 6: ein zweites Ausführungsbeispiel der Realisierung eines positionssensitiven Identifikators.

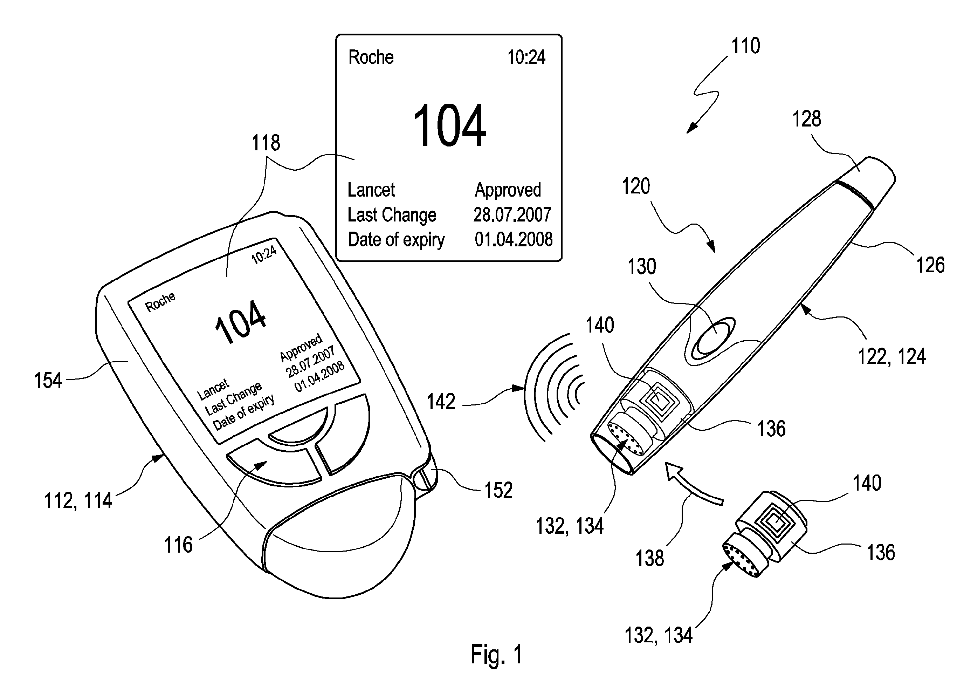

In Figur 1 ist ein erstes, einfaches Ausführungsbeispiel eines medizinischen Systems 110 dargestellt. Es handelt sich um ein medizinisches System 110, welches für die Diabetes-Kontrolle eingesetzt werden kann und welches in diesem Ausführungsbeispiel zwei grundsätzlich unabhängig voneinander einsetzbare Einzelkomponenten umfasst. So umfasst das medizinische System 110 zunächst ein Messgerät 112, welches in dem medizinischen System 110 als zentrale Steuereinheit 114 fungiert. In dem hier dargestellten Ausführungsbeispiel handelt es sich bei dem Messgerät 112 beispielsweise um ein Blutglukose-Messgerät, welches mittels eines (in Figur 1 nicht dargestellten) Testelements und beispielsweise eines optischen und/oder elektrochemischen Messverfahrens eine Blutglukose-Konzentration in einem Blutstropfen ermitteln kann. Das Messgerät 112 verfügt über Bedienelemente 116 zur Bedienung der Systemfunktionen, über eine (in Figur 1 nicht zu erkennende) Ansteuer- und Auswerteelektronik zur Auswertung der Teststreifen-Messung sowie über ein Anzeigeelement 118, welches zur Verdeutlichung in Figur 1 rechts neben dem Messgerät 112 nochmals in vergrößerter Darstellung gezeigt ist. Weiterhin kann das Messgerät 112 zusätzliche Komponenten umfassen, wie beispielsweise eine oder mehrere Schnittstellen, ein Magazin zur Aufnahme von Testelementen oder ähnliches.

Als zweite Komponente umfasst das medizinische System 110 gemäß Figur 1 eine invasive Einheit 120, welche in diesem Ausführungsbeispiel ausgestaltet ist, um eine Probengewinnungsfunktion zur Gewinnung einer flüssigen Probe, insbesondere eines Bluttropfens, auszuüben. Insofern ist die Funktion der invasiven Einheit 120 unabhängig von der Funktion des Messgerätes 112, auch wenn diese insgesamt das medizinische System 110 zur Diabetes-Kontrolle bilden.

Die invasive Einheit 120 umfasst ein Peripheriegerät 122 in Form einer Stechhilfe 124. Die Stechhilfe 124 verfügt über einen im Inneren eines Gehäuses 126 angeordneten Federmechanismus, welcher über einen Spannknopf 128 spannbar und über einen Auslöseknopf 130 auslösbar ist.

Weiterhin umfasst die invasive Einheit 120 in diesem Ausführungsbeispiel mehrere invasive Verbrauchsmittel 132, welche in diesem Ausführungsbeispiel in Form von Lanzetten 134 ausgestaltet sind. Die Lanzetten 134 sind in einem trommelförmigen Magazin 136 zusammengefasst, wobei jede der Lanzetten 134 in einer separaten Lanzettenkammer angeordnet ist. Das Magazin 136 wird in das Gehäuse 126 der Stechhilfe 124 eingefügt, so dass für jeden Stechvorgang eine Lanzette 134 einsetzbar ist. Beispielsweise kann es sich bei dem dargestellten Magazin 136 um ein kommerziell erhältliches Magazin vom Typ Accu-Chek Multicix Safety Drum handeln. Dabei ist in Figur 1 das Magazin 136 einmal im nicht-eingebauten Zustand und einmal im in die Stechhilfe 124 eingesetzten Zustand dargestellt, wobei der Vorgang des Einlegens in Figur 1 symbolisch mit dem Pfeil 138 bezeichnet ist.

Weiterhin sind die invasiven Verbrauchsmittel 132 in dem in Figur 1 dargestellten Ausführungsbeispiel mit einem Identifikator 140 ausgestattet. Dieser Identifikator 140 ist in Figur 1 lediglich symbolisch dargestellt und wird hier als RFID-Chip symbolisiert. Dieser Identifikator 140 ist somit elektronisch auslesbar und ist in der Lage, mindestens eine Information zu speichern. Diese Information kann berührungslos auf elektronischem Wege von dem Messgerät 112 abgefragt bzw. ausgelesen werden, was in Figur 1 symbolisch mit der Bezugsziffer 142 bezeichnet ist. Beispielsweise kann diese elektronische Informationsübertragung über Radiowellen erfolgen, beispielsweise Radiowellen im Megahertz- oder Gigahertz-Bereich, wie es für derartige Identifikatoren 140 üblich ist.

Weiterhin wird darauf hingewiesen, dass in dem in Figur 1 dargestellten Ausführungsbeispiel ein einzelner Identifikator 140 an dem Magazin 136 angebracht ist. Insofern wird zwischen den in dem Magazin 136 zusammengefassten invasiven Verbrauchsmitteln 132 in Form der Lanzetten 134 und dem Magazin 136 selbst im Folgenden nicht unterschieden. Es sei jedoch darauf hingewiesen, dass auch die einzelnen Lanzetten 134 bzw. invasiven Verbrauchsmittel 132 jeweils gekennzeichnet sein können, beispielsweise ähnlich zu der Lanzettenmarkierung, welche in US 2004/0138688 A1 dargestellt ist, jedoch mit elektronisch auslesbaren Identifikatoren. Auch Kombinationen dieser Ausgestaltungen sind denkbar, also eine Ausgestaltung, bei welcher alle invasiven Verbrauchsmittel 132 mit Identifikatoren ausgestaltet sind und bei welcher zusätzlich das Magazin 136 einen derartigen Identifikator 140 aufweist. Weiterhin kann - wie oben beschrieben - auch die Stechhilfe 124 selbst einen Identifikator 140 aufweisen.

Auf diese Weise kann durch die zentrale Steuereinheit 114 beispielsweise erkannt werden, dass sich eine Stechhilfe 124 und/oder invasive Verbrauchsmittel 132 in einer Ausleseentfemung befinden. Weiterhin kann erkannt werden, um welche Arten von Verbrauchsmitteln 132 es sich handelt, was neue Funktionen und eine Fälschungssicherung ermöglicht. Für diese neuen Funktionen sei exemplarisch auf die obige Beschreibung verwiesen. Exemplarisch ist in Figur 1 dargestellt, dass beispielsweise vom Messgerät 112 erkannt wird, dass es sich um autorisierte Verbrauchsmittel 132 handelt. Dies kann beispielsweise für eine entsprechende Anzeige auf dem Anzeigeelement 118 genutzt werden, wie dies in Figur 1 symbolisch angedeutet ist ("Lancet approved"). Weiterhin kann das Datum des letzten Wechsels des Verbrauchsmaterials angezeigt werden ("last change") und es kann ein Verfallsdatum ("date of expiry") des Verbrauchsmittels angezeigt werden. Weitere mögliche Ausführungsbeispiele sind in der obigen Beschreibung aufgeführt.

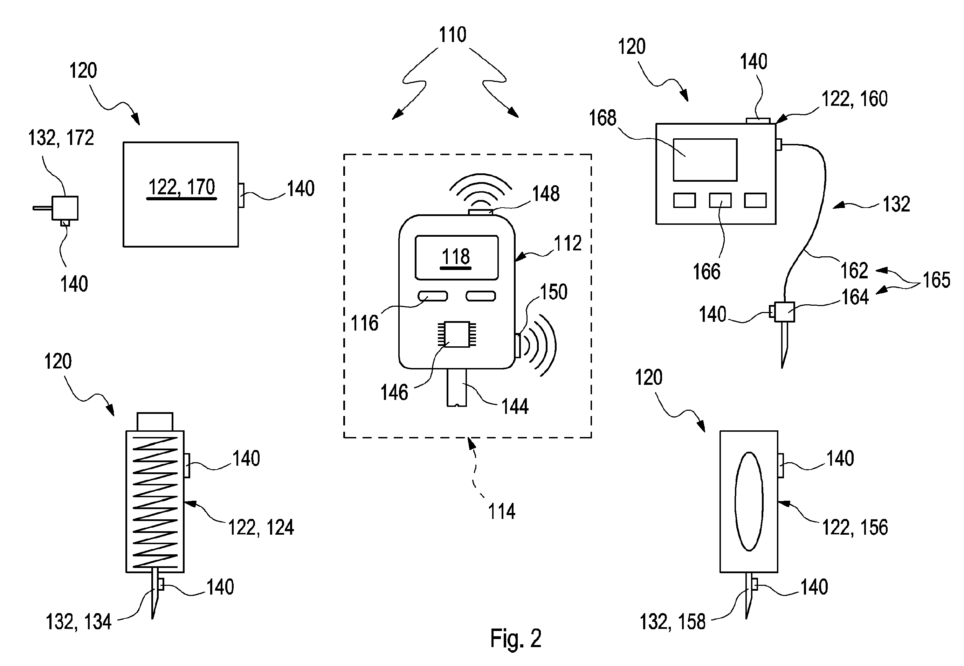

In Figur 2 ist eine Erweiterung des oben beschriebenen medizinischen Systems 110 symbolisch dargestellt. In diesem Fall umfasst das medizinische System wiederum eine zentrale Steuereinheit 114 in Form eines Messgerätes 112. Das Messgerät 112 kann beispielsweise analog zur Beschreibung gemäß Figur 1 ausgestaltet sein, kann über ein Anzeigeelement 118 und Bedienelement 116 verfügen und kann beispielsweise ausgestaltet sein, um mittels eines Teststreifens 144 oder mittels eines auf andere Weise ausgestalteten Testelements (zum Beispiel eines Testbandes) eine Analytkonzentration, beispielsweise eine Blutglukose-Konzentration, zu bestimmen und diese als diagnostischen Messwert auf dem Anzeigeelement 118 darzustellen.

Weiterhin ist in Figur 2 symbolisch eine Datenverarbeitungseinrichtung 146 dargestellt, welche beispielsweise zur elektronischen Verarbeitung diagnostischer Messwerte und/oder zur Ausübung anderer Systemfunktionen (wie zum Beispiel der Auswertung der von dem Identifikator 140 übertragenen Informationen) genutzt werden kann. Weiterhin verfügt das Messgerät 112 in dem in Figur 2 dargestellten Ausführungsbeispiel über zwei Schnittstellen, welche lediglich symbolisch dargestellt sind. Eine erste Schnittstelle 148 dient dem Datenaustausch mit externen Geräten, beispielsweise externen Computern. Diese erste Schnittstelle 148, welche beispielsweise als Infrarot-Schnittstelle ausgestaltet sein kann, kann somit beispielsweise genutzt werden, um Software-Aktualisierungen auf dem Messgerät 112 vorzunehmen und/oder um medizinische Messdaten an einen externen Computer (beispielsweise einen Arztcomputer und/oder einen Patientencomputer) zu übermitteln. Daneben verfügt das Messgerät 112 über eine zweite Schnittstelle 150, über welche berührungslos und auf elektronischem Wege Informationen abgefragt werden können, welche in Identifikatoren 140 der invasiven Einheiten 120 abgespeichert sind. Diese zweite Schnittstelle 150 ist vorzugsweise eine Funkschnittstelle. Beide Schnittstellen 148, 150 können jedoch auch zu einer einzelnen Schnittstelle zusammengefasst sein.

In Figur 2 ist symbolisch die zentrale Steuereinheit 114 durch die gestrichelte Linie als abgeschlossene Einheit dargestellt. Hierdurch soll symbolisiert werden, dass diese abgeschlossene Einheit funktionell autark arbeiten kann, wobei jedoch dieser funktionellen Einheit 114 auch weitere Komponenten, wie beispielsweise eine Spenderbox zur Bereitstellung von Teststreifen 144 oder ähnliche Komponenten, zugeordnet sein können.

Neben dieser zentralen Steuereinheit 114 umfasst das medizinische System 110 in dem in Figur 2 dargestellten Beispiel - im Unterschied zu Figur 1 - vier invasive Einheiten 120. Diese invasiven Einheiten 120 sind funktionell vorzugsweise unabhängig von der zentralen Steuereinheit 114 und sind auch untereinander vorzugsweise unabhängig. Diese funktionelle Unabhängigkeit, welche durch die gestrichelte Linie 114 dargestellt ist, bedeutet jedoch nicht, dass diese Komponenten nicht in räumliche Nähe zur zentralen Steuereinheit 114 angeordnet werden können. Dies wird aus Figur 1 deutlich, in welcher am Messgerät 112 eine Koppelstelle 152 erkennbar ist, über welche die Stechhilfe 124 an einem Gehäuse 154 ankoppelbar ist. In diesem angekoppelten Zustand bilden die zentrale Steuereinheit 114 und die invasive Einheit 120 in Form der Stechhilfe 124 zwar ein mechanisch zusammengefasstes System. Die Systemfunktionen der zentralen Steuereinheit 114 (Messen und Anzeigen) und der invasiven Einheit 120 (Probengewinnung) sind jedoch nach wie vor klar getrennt.

Bei dem medizinischen System 110 in Figur 2 umfassen die invasiven Einheiten 120 wiederum eine Stechhilfe 124 als Peripheriegerät 122, welche mit invasiven Verbrauchsmitteln 132 in Form von Lanzetten 134 zusammenwirkt. Für die Beschreibung der Stechhilfe 124 kann weitgehend auf die Figur 1 verwiesen werden.

Weiterhin umfasst das medizinische System 110 eine invasive Einheit 120 in Form eines als Insulin-Pen 156 ausgestalteten Peripheriegerätes 122. Dieser Insulin-Pen 156 ist in Figur 2 lediglich symbolisch dargestellt und wirkt mit einem invasiven Verbrauchsmittel 132 in Form einer Kanüle 158 zusammen. Der Insulin-Pen 156 ist ausgestaltet, um einem Patienten die Injektion einer voreingestellten Medikationsmenge an Insulin zu ermöglichen.

Weiterhin umfasst das medizinische System 110 ein als Insulinpumpe 160 ausgestaltetes Peripheriegerät 122, welches mit einem Verbrauchsmittel 132 in Form eines Schlauches 162 und einer Kanüle 164 zusammenwirkt. Schlauch 162 und Kanüle 164 sind üblicherweise als gemeinsame, entsorgbare Einheit ausgestaltet, welche in Figur 2 als Katheter 165 bezeichnet ist. Die Insulinpumpe 160 ermöglicht es somit einem Patienten, über eine (in Figur 2 nicht dargestellte) Pumpe einem Patienten eine mittels Bedienelementen 166 voreingestellte und auf einem Anzeigeelement 168 dargestellte Menge an Insulin aus einem Vorratsgefäß zuzuführen.

Als vierte invasive Einheit 120 umfasst das medizinische System 110 ein Peripheriegerät 122 in Form einer Messeinheit 170, welche mit einem Verbrauchsmittel 132 in Form eines Subkutansensors 172 zusammenwirkt, um eine kontinuierliche Überwachung beispielsweise eines Blutglukosewertes in Gewebeschichten eines Patienten zu gewährleisten.

Es sei darauf hingewiesen, dass die in Figur 2 dargestellten invasiven Einheiten 120 lediglich exemplarisch ausgewählt sind gemäß üblichen, in der Diabetes-Überwachung verwendeten Komponenten. Naturgemäß kann das medizinische System 110 jedoch auch weitere oder andere Arten von Komponente umfassen.

In dem in Figur 2 dargestellten Ausführungsbeispiel sind sowohl die invasiven Verbrauchsmittel 132 als auch die Peripheriegeräte 122 jeweils mit elektronischen Identifikatoren 140 ausgestaltet. Wie oben beschrieben, ist jedoch auch eine andere Ausgestaltung möglich, beispielsweise indem ausschließlich die invasiven Verbrauchsmittel 132 oder die Peripheriegeräte 122 derart ausgestattet sind. Über die zweite Schnittstelle 150 kann die zentrale Steuereinheit 114 somit Informationen dieser elektronischen Identifikatoren 140 abfragen.

Als Identifikatoren 140 kommen überwiegend sehr preiswerte und kleine Varianten elektronisch auslesbarer Identifikatoren in Betracht. Die Information der Identifikatoren 140 sollte auslesbar sein, wenn sich die invasiven Einheiten 120 in der Nähe des Messgeräts 112 befinden, beispielsweise wenn die Stechhilfe 124 an das Messgerät 112 angekoppelt ist bzw. mit diesem verbunden ist. Auch eine Anordnung in räumlicher Nähe, beispielsweise in einem Abstand unterhalb von 50 cm, ist jedoch denkbar.

Als elektronische Identifikatoren 140 kommen beispielsweise RFID-Chips der Fa. Hitachi in Japan in Betracht, welche Silicium-Chips mit Abmessungen unterhalb von 0,4 mm Kantenlänge inklusive Antenne aufweisen. Typische Arbeitsfrequenzen liegen hier bei 2,45 GHz, Speicherkapazitäten bei 128 Bit. Derartige RFID-Chips sind potentiell äußerst preiswert, da diese ursprünglich für die Verwendung in Banknoten entwickelt wurden. Weiterhin kommen auch beispielsweise RFID-Chips vom Typ "mic3^{®}TAG" der microsensys GmbH, Deutschland, in Betracht, welche Kantenlängen von ca. 1,6 mm x 1 mm, Arbeitsfrequenzen im Bereich von 13,56 MHz und Speicherkapazitäten von 64 Bit ROM aufweisen. Auch andere Arten von RFID-Chips sind jedoch einsetzbar.

Als weitere Möglichkeit eines Ausführungsbeispiels der Identifikatoren 140 sei auf die sogenannten "chipless identifier" der Acreo AB, Schweden, hingewiesen. Hier wird die gespeicherte Information durch einen aufgedruckten Barcode aus leitfähiger Tinte codiert (das sogenannte HidE-Prinzip). Es handelt sich hierbei um ein kostengünstiges Verfahren, was leicht in bisherigen Produktprozessen integrierbar ist und bei welchem ca. 10 bis 30 Bit Information in den Identifikator 140 eingebracht und ausgelesen werden können. Die Größe der einspeicherbaren Informationen richtet sich dabei im Wesentlichen nach der Größe des elektronischen Barcodes, also insbesondere auch nach der Größe des Verbrauchsmittels 132.

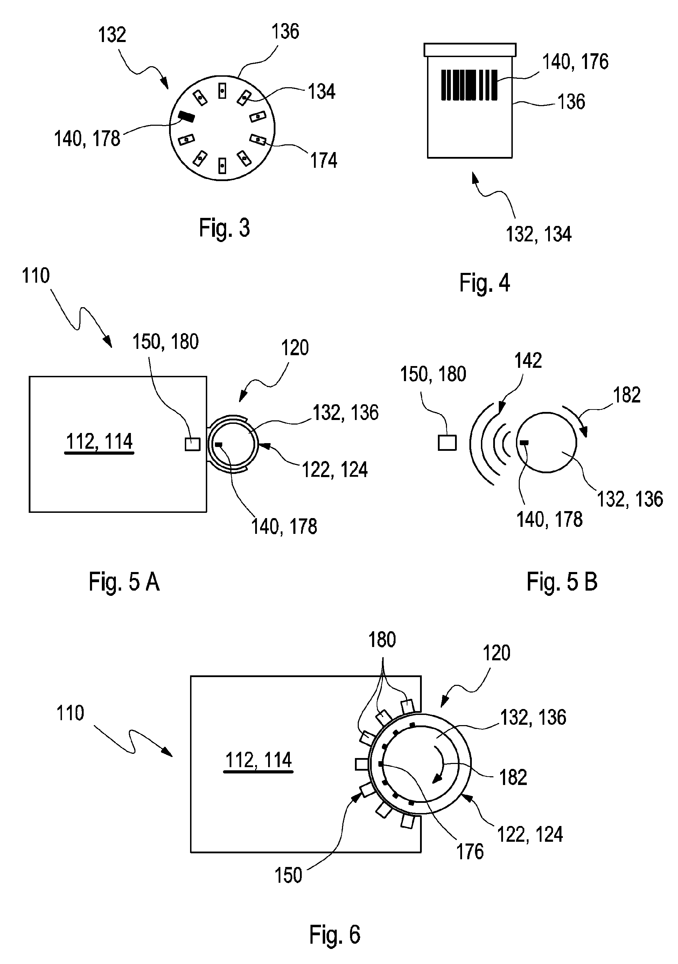

Der Identifikator 140 kann auf unterschiedliche Weisen an der invasiven Einheit 120 angeordnet sein. Beispiele derartiger Anordnungen beziehungsweise Anbringungen sind in Figur 3 und in Figur 4 dargestellt. So zeigt Figur 3 ein Ausführungsbeispiel invasiver Verbrauchsmittel 132, welche wiederum in Form von Lanzetten 134 ausgestaltet sind. Diese Lanzetten sind, analog zum Ausführungsbeispiel in Figur 1, in einem trommelförmigen Magazin 136 angeordnet, wobei die einzelnen Lanzetten 134 in Lanzettenhohlräumen 174 angeordnet sind. Diese Lanzettenhohlräume 174 sind rotationssymmetrisch radial nach außen weisend in dem Trommelmagazin 136 angeordnet.

Dabei ist in Figur 3 eine Anordnung gewählt, bei der ein Lanzettenhohlraum 174 nicht mit einer Lanzette 134 ausgefüllt ist. Stattdessen wird in diesem Ausführungsbeispiel ein Identifikator 140 in den Lanzettenhohlraum 174 eingebracht.

In Figur 4 ist hingegen ein Magazin 136 dargestellt, bei welchem ein Identifikator 140 von außen auf die Außenwand des Magazins 136 aufgebracht ist. Im Gegensatz zur Figur 3, in welcher das Magazin 136 in Draufsicht gezeigt ist, ist in Figur 4 das Magazin 136 in einer Darstellung mit Ansicht von der Seite gezeigt. Während in der Ausführungsform gemäß Figur 3 beispielsweise ein RFID-Chip 178 als Identifikator 140 einsetzbar ist, wird in der Ausführungsform gemäß Figur 4 vorzugsweise als Identifikator 140 ein leitfähiger Barcode 176 (chipless identifier) auf die Seitenwand des Magazins 136 aufgedruckt, aufgeklebt oder auf andere Weise aufgebracht. Verschiedene andere Ausgestaltungen der Anbringung des Identifikators 140 sind möglich.

In den Figuren 5A bis 6 sind verschiedene Möglichkeiten dargestellt, die Identifikatoren 140 mittels der zentralen Steuereinheit 114 auszulesen. Dabei zeigen die Figuren 5A und 5B ein Ausführungsbeispiel, welches dem medizinischen System 110 gemäß Figur 1 entspricht. Dabei ist die Stechhilfe 124 in dem gezeigten Ausführungsbeispiel an dem Messgerät 112 angekoppelt, und in der Stechhilfe 124 sind invasive Verbrauchsmittel 132 mit einem Magazin 136 angeordnet. In dem Magazin ist - wie beispielsweise in Figur 3 gezeigt - ein RFID-Chip 178 als Identifikator 140 in einen (nicht dargestellten) Lanzettenhohlraum 174 eingefügt. Das Messgerät 112 verfügt über eine zweite Schnittstelle 150 in Form einer Antenne 180, über welche die Information des RFID-Chips 178 ausgelesen werden kann. Dabei kann diese zweite Schnittstelle 150 derart ausgestaltet sein, dass diese nicht nur das Signal des RFID-Chips 178 selbst bezüglich dessen Inhalt abfragt, sondern auch eine Signalintensität registriert.

Während in Figur 5A das gesamte medizinische System 110 symbolisch dargestellt ist, ist in Figur 5B lediglich der Fall symbolisch und stark vereinfacht dargestellt, in welchem sich das Magazin 136 relativ zum Messgerät 112 (welches in Figur 5B nicht dargestellt ist) dreht. Durch diese Drehung, welche in Figur 5B mit der Bezugsziffer 182 bezeichnet ist, wird das Signal des Identifikators 140 (in Figur 5B durch das "Auslesen" 142 bezeichnet) schwächer. Diese Abschwächung kann vom Messgerät 112 registriert werden, so dass hieraus auf eine Position des Identifikators 140 und somit auf eine Winkelstellung des Trommelmagazins 136 geschlossen werden kann. In diesem Fall handelt es sich also um einen positionssensitiven Identifikator 140, welcher asymmetrisch an den Verbrauchsmitteln 132 bzw. deren Magazin 136 angeordnet ist, so dass mittels des Messgerätes 112 nicht nur die Information des Identifikators 140 ausgelesen werden kann, sondern aus der Signalstärke oder anderen Signaleigenschaften auch auf eine Positionierung beziehungsweise Orientierung der Verbrauchsmittel 132 geschlossen werden kann.

In Figur 6 ist ein anderes Ausführungsbeispiel eines positionssensitiven Identifikators 140 schematisch dargestellt. Wieder entspricht das dargestellte medizinische System 110 beispielsweise dem medizinischen System 110 gemäß Figur 1, so dass wiederum auf die obige Beschreibung verwiesen werden kann.

In diesem Ausführungsbeispiel entspricht jedoch der Identifikator 140 dem Ausführungsbeispiel gemäß Figur 4 und umfasst einen umfangsseitig auf dem Magazin 136 aufgebrachten leitfähigen Barcode 176. Die zweite Schnittstelle 150 des Messgerätes 112 umfasst mehrere einzelne Antennen 180, welche insgesamt die Positionierung der Streifen oder Flächen des leitfähigen Barcodes 176 bestimmten können. Ähnlich zu Plattenkondensatoren ändert sich beispielsweise ein elektrisches Feld zwischen diesen Antennen 180 und den Streifen des leitfähigen Barcodes 176, was zur Bestimmung der Positionierung der Magazintrommel 136 genutzt werden kann. Wird das Magazin 136 gedreht (Bezugsziffer 182 in Figur 6), so verändert sich das Signal an dieser zweiten Schnittstelle 150, woraus wiederum auf die Orientierung der Verbrauchsmittel 132 geschlossen werden kann.

### Bezugszeichenliste

- 110: medizinisches System
- 112: Messgerät
- 114: zentrale Steuereinheit
- 116: Bedienelement
- 118: Anzeigeelement
- 120: invasive Einheit
- 122: Peripheriegerät
- 124: Stechhilfe
- 126: Gehäuse Stechhilfe
- 128: Spannknopf
- 130: Auslöseknopf
- 132: invasives Verbrauchsmittel
- 134: Lanzetten
- 136: Magazin
- 138: Einlegen Magazin in Stechhilfe
- 140: Identifikator
- 142: Auslesen des Identifikators
- 144: Teststreifen
- 146: Datenverarbeitungseinrichtung
- 148: erste Schnittstelle
- 150: zweite Schnittstelle
- 152: Koppelstelle
- 154: Gehäuse Messgerät
- 156: Insulin-Pen
- 158: Kanüle
- 160: Insulinpumpe
- 162: Schlauch
- 164: Kanüle
- 165: Katheter
- 166: Bedienelement
- 168: Anzeigeelement
- 170: Messeinheit
- 172: Subkutansensor
- 174: Lanzettenhohlraum

- 176: leitfähiger Barcode
- 178: RFID-Chip
- 180: Antenne
- 182: Drehung

## Patentansprüche

1. Medizinisches System (110), umfassend eine zentrale Steuereinheit (114), welche eingerichtet ist, um mindestens einen diagnostischen Messwert mittels eines Anzeigeelements (118) anzuzeigen, weiterhin umfassend mindestens eine invasive Einheit (120), wobei die invasive Einheit (120) mindestens ein invasives Verbrauchsmittel (132) umfasst, wobei das invasive Verbrauchsmittel (132) eingerichtet ist, um invasiv in ein Gewebe eines Patienten einzugreifen, wobei die invasive Einheit (120) mindestens einen berührungslos auslesbaren elektronischen Identifikator (140) zur Speicherung mindestens einer Information aufweist, **dadurch gekennzeichnet, dass** die zentrale Steuereinheit (114) eingerichtet ist, um die mindestens eine Information des elektronischen Identifikators (140) elektronisch auszulesen.

2. Medizinisches System (110) nach dem vorhergehenden Anspruch, wobei die zentrale Steuereinheit (114) und die invasive Einheit (120) funktionell unabhängig voneinander betätigbar sind.

3. Medizinisches System (110) nach einem der beiden vorhergehenden Ansprüche, wobei die zentrale Steuereinheit (114) mindestens eine Messfunktion zur Ermittlung des diagnostischen Messwerts aufweist, wobei die invasive Einheit (120) mindestens eine der folgenden Funktionen aufweist: eine Probengewinnungsfunktion, insbesondere eine Lanzettenfunktion; eine Medikationsfunktion, insbesondere eine Dosierungsfunktion; eine analytische und/oder diagnostische Funktion, wobei die analytische und/oder diagnostische Funktion der invasiven Einheit (120) funktionell unabhängig ist von der Messfunktion der zentralen Steuereinheit (114).

4. Medizinisches System (110) nach dem vorhergehenden Anspruch, wobei die invasive Einheit (120) ausschließlich mechanische Funktionen aufweist.

5. Medizinisches System (110) nach einem der vorhergehenden Ansprüche, wobei die zentrale Steuereinheit (114) mindestens eine optische Messeinheit und/oder elektrochemische Messeinheit aufweist, welche eingerichtet ist, um mittels mindestens eines Testelements (144) eine Konzentration eines Analyten in einer Körperflüssigkeit zu ermitteln.

6. Medizinisches System (110) nach einem der vorhergehenden Ansprüche, wobei die invasive Einheit (120) mindestens eine der folgenden Einheiten aufweist:
- ein als Stechhilfe (124) ausgestaltetes Peripheriegerät (122), wobei die Stechhilfe (124) ausgestaltet ist, um eine Hautpartie eines Patienten mittels eines als Lanzette (134) ausgestalteten invasiven Verbrauchsmittels (132) zu perforieren;
- ein als Medikationsstift ausgestaltetes Peripheriegerät (122), insbesondere einen Insulin-Pen (156), wobei der Medikationsstift ausgestaltet ist, um einem Patienten mittels eines als Kanüle (158) ausgestalteten invasiven Verbrauchsmittels (132) eine Dosis eines Medikaments zu injizieren;
- ein als Medikationspumpe ausgestaltetes Peripheriegerät (122), insbesondere eine Insulinpumpe (160), wobei die Medikationspumpe ausgestaltet ist, um einem Patienten mittels eines als Katheter (165) ausgestalteten invasiven Verbrauchsmittels (132) eine Dosis eines Medikaments zu injizieren;
- ein als Messeinheit (170) ausgestaltetes Peripheriegerät (122), wobei die Messeinheit (170) ausgestaltet ist, um mittels eines invasiven Verbrauchsmittels (132), welches einen in einem Körpergewebe eines Patienten implantierten Subkutansensor (172) umfasst, eine Konzentration eines Analyten, insbesondere eines Metaboliten, in einer Körperflüssigkeit zu ermitteln.

7. Medizinisches System (110) nach einem der vorhergehenden Ansprüche, wobei die zentrale Steuereinheit (114) eingerichtet ist, um entsprechend der ausgelesenen Information einen akustischen Hinweis und/oder einen optischen Hinweis und/oder einen haptischen Hinweis, insbesondere durch eine Vibration, an den Benutzer zu übermitteln.

8. Medizinisches System (110) nach einem der vorhergehenden Ansprüche, wobei die Information mindestens eine der folgenden Informationen umfasst:
- eine Information über einen Hersteller der invasiven Einheit (120) und/oder des invasiven Verbrauchsmittels (132);
- eine Information über ein Herstelldatum der invasiven Einheit (120) und/oder des invasiven Verbrauchsmittels (132);
- eine Information über die Art der invasiven Einheit (120) und/oder des invasiven Verbrauchsmittels (132), insbesondere eine Chargennummer;
- eine individuelle Seriennummer;
- eine Anzahl von in der invasiven Einheit (120) enthaltenen invasiven Verbrauchsmitteln (132);
- ein Haltbarkeitsdatum der invasiven Einheit (120) und/oder des invasiven Verbrauchsmittels (132).

9. Medizinisches System (110) nach einem der vorhergehenden Ansprüche, wobei die invasive Einheit (120) zusätzlich mindestens ein Peripheriegerät (122) aufweist, wobei das Peripheriegerät (122) eingerichtet ist, um mit dem invasiven Verbrauchsmittel (132) zusammenzuwirken.

10. Medizinisches System (110) nach dem vorhergehenden Anspruch, wobei das invasive Verbrauchsmittel (132) mindestens einen ersten berührungslos auslesbaren elektronischen Identifikator (140) zur Speicherung mindestens einer ersten Information aufweist, wobei das Peripheriegerät (122) mindestens einen zweiten berührungslos auslesbaren elektronischen Identifikator (140) zur Speicherung mindestens einer zweiten Information aufweist.

11. Medizinisches System (110) nach dem vorhergehenden Anspruch, wobei die zentrale Steuereinheit (114) eingerichtet ist, um die mindestens eine erste Information und die mindestens eine zweite Information auszulesen.

12. Medizinisches System (110) nach einem der beiden vorhergehenden Ansprüche, wobei die zentrale Steuereinheit (114) eingerichtet ist, um die erste Information mit mindestens einer ersten Sollinformation zu vergleichen und um die zweite Information mit mindestens einer zweiten Sollinformation zu vergleichen.

13. Medizinisches System (110) nach einem der vorhergehenden Ansprüche, wobei mehrere invasive Verbrauchsmittel (132) in einem Verbrauchsmittelmagazin (136) der invasiven Einheit (120) zusammengefasst sind.

14. Medizinisches System (110) nach einem der vorhergehenden Ansprüche, wobei die zentrale Steuereinheit (114) eingerichtet ist, um entsprechend der Information mindestens eine der folgenden Aktionen durchzuführen:
- die Information wird für eine spätere Auswertung gespeichert;
- die Information wird bei einer Fehlerdiagnose ausgewertet;
- die Information wird an einen Benutzer bereitgestellt;
- wenn die Information von einem Sollwert oder Sollbereich abweicht, wird eine Warnung an einen Benutzer ausgegeben oder eine Warninformation in einem Datenspeicher abgelegt;
- wenn die Information von einem Sollwert oder Sollbereich abweicht, wird mindestens eine Gerätefunktion der zentralen Steuereinheit (114) gesperrt;
- es wird ermittelt, wann das invasive Verbrauchsmittel (132) der invasiven Einheit (120) zuletzt gewechselt wurde;
- ein Hersteller der invasiven Einheit (120) wird ermittelt;
- ein Hersteller des invasiven Verbrauchsmittels (132) wird ermittelt.

15. Medizinisches System (110) nach einem der vorhergehenden Ansprüche, wobei die zentrale Steuereinheit (114) mindestens eine erste Schnittstelle (148) zum Datenaustausch mit einem externen Datenverarbeitungsgerät umfasst und wobei die zentrale Steuereinheit (114) mindestens eine zweite Schnittstelle (150) zum Austausch der Information mit dem Identifikator (140) umfasst.

16. Medizinisches System (110) nach einem der vorhergehenden Ansprüche, wobei der Identifikator (140) einen passiven Identifikator (140) ohne eigene Energieversorgung umfasst.

17. Medizinisches System (110) nach einem der vorhergehenden Ansprüche, wobei der Identifikator (140) mindestens einen der folgenden Identifikatoren (140) umfasst:
- einen elektronischen Barcode, insbesondere einen leitfähigen Barcode (176);
- einen veränderlichen Identifikator (176; 178);
- einen positionssensitiven Identifikator (176; 178) zur Erkennung einer Position und/oder Orientierung des invasiven Verbrauchsmittels (132) innerhalb der invasiven Einheit (120);
- einen Hochfrequenzchip (178).

18. Verfahren zur Überwachung eines medizinischen Systems (110), insbesondere eines medizinischen Systems (110) nach einem der vorhergehenden Ansprüche, wobei das medizinische System (110) eine zentrale Steuereinheit (114) umfasst, welche eingerichtet ist, um mindestens einen diagnostischen Messwert mittels eines Anzeigeelements (118) anzuzeigen, weiterhin umfassend mindestens eine invasive Einheit (120), wobei die invasive Einheit (120) mindestens ein invasives Verbrauchsmittel (132) umfasst, wobei das invasive Verbrauchsmittel (132) eingerichtet ist, um invasiv in ein Gewebe eines Patienten einzugreifen, wobei die Invasive Einheit (120) mindestens einen berührungslos auslesbaren elektronischen Identifikator (140) zur Speicherung mindestens einer Information aufweist, wobei die zentrale Steuereinheit (114) die mindestens eine Information des elektronischen Identifikators (140) ausliest.

19. Verfahren nach dem vorhergehenden Anspruch, wobei von der zentralen Steuereinheit (114) anhand der Information ermittelt wird, ob das invasive Verbrauchsmittel (132) bereits zuvor in dem medizinischen System (110) verwendet wurde.

20. Verfahren nach einem der vorhergehenden Verfahrensansprüche, wobei von der zentralen Steuereinheit (114) anhand der Information ermittelt wird, ob ein Haltbarkeitsdatum der invasiven Einheit (120) und/oder des invasiven Verbrauchsmittels (132) abgelaufen ist.

21. Verfahren nach einem der vorhergehenden Verfahrensansprüche, wobei die Information für eine spätere Auswertung in einem Datenspeicher der zentralen Steuereinheit (114) gespeichert wird.

22. Verfahren nach dem vorhergehenden Anspruch, wobei die Information im Rahmen einer Fehlerdiagnose und/oder einer Reklamation ausgewertet wird.

23. Verfahren nach einem der vorhergehenden Verfahrensansprüche, wobei von der zentralen Steuereinheit (114) anhand der Information ermittelt wird, ob die invasive Einheit (120) und/oder des invasive Verbrauchsmittel (132) für die Verwendung in dem medizinischen System (110) geeignet sind.

24. Verfahren nach einem der vorhergehenden Verfahrensansprüche, wobei von der zentralen Steuereinheit (114) anhand der Information ermittelt wird, wie viele invasive Verbrauchsmittel (132) die invasive Einheit (120) umfasst.

25. Verfahren nach einem der vorhergehenden Verfahrensansprüche, wobei von der zentralen Steuereinheit (114) anhand der Information ermittelt wird, wie viele unbenutzte invasive Verbrauchsmittel (132) die invasive Einheit (120) umfasst.

26. Verfahren nach einem der vorhergehenden Verfahrensansprüche, wobei von der zentralen Steuereinheit (114) anhand der Information ermittelt wird, ob das invasive Verbrauchsmittel (132) bereits verwendet worden ist und wobei, wenn festgestellt wird, dass dies der Fall ist, ein Benutzer zur Verwendung eines neuen invasiven Verbrauchsmittels (132) aufgefordert wird und/oder bewirkt wird, dass die invasive Einheit (120) ein neues invasives Verbrauchsmittel (132) verwendet.

27. Verfahren nach einem der vorhergehenden Verfahrensansprüche, wobei von der zentralen Steuereinheit (114) eine Information über eine Identität der zentralen Steuereinheit (114) in dem Identifikator (140) gespeichert wird.

28. Verfahren nach einem der vorhergehenden Verfahrensansprüche, wobei von der zentralen Steuereinheit (114) anhand der Information ermittelt wird, ob das invasive Verbrauchsmittel (132) in der invasiven Einheit (120) korrekt positioniert ist.

29. Verfahren nach einem der vorhergehenden Verfahrensansprüche, wobei von der zentralen Steuereinheit (114) anhand der Information ermittelt wird, wann der letzte Wechsel des invasiven Verbrauchsmittels (132) stattgefunden hat.

30. Verfahren nach dem vorhergehenden Anspruch, wobei bei überschreiten einer Maximaldauer seit dem letzten Wechsel des invasiven Verbrauchsmittels (132) ein Warnhinweis an einen Benutzer ausgegeben wird.

31. Verfahren nach einem der vorhergehenden Verfahrensansprüche, wobei das invasive Verbrauchsmittel (132) mindestens eine Lanzette (134) umfasst, wobei die invasive Einheit (120) ein Peripheriegerät (122) in Form einer Stechhilfe (124) umfasst, wobei von der zentralen Steuereinheit (114) anhand der Information ermittelt wird, welche Einstechtiefe an der Stechhilfe (124) eingestellt ist.
